# EUROPEAN PATENT SPECIFICATION

(11) **EP 3 707 120 B1**
(45) Date of publication and mention of the grant of the patent: **05.01.2022**
(21) Application number: 18799533.7
(22) Date of filing: 07.11.2018
(51) Int. Cl.: C07C 47/453, C11B 9/00

(54) **ALDEHYDES FOR USE AS ODORANTS**
ALDEHYDE ZUR VERWENDUNG ALS DUFTSTOFFE
ALDÉHYDES DESTINÉS À ÊTRE UTILISÉS EN TANT QUE MATIÈRES ODORANTES

(30) Priority: 08.11.2017 GB 201718491
(43) Date of publication of application: 16.09.2020
(73) Proprietor: Givaudan SA, 1214 Vernier (CH)
(72) Inventor: LINIGER, Marc, 8424 Embrach (CH)
(74) Representative: Global Patents
(86) International application number: PCT/EP2018/080526
(87) International publication number: WO 2019/092056

(56) References cited:
- WO-A1-2014/180952
- BÉAT WINTER ET AL: "Synthesis and Odor Properties of Substituted Indane-2-carboxaldehydes. Discovery of a New Floral (Muguet) Fragrance Alcohol", HELVETICA CHIMICA ACTA, vol. 88, no. 12, 27 December 2005 (2005-12-27), pages 3118-3127, XP055542622, CH ISSN: 0018-019X, DOI: 10.1002/hlca.200590251

## Description

The present invention relates to novel 2-methyl-2,3-dihydro-1H-indene-2-carbaldehyde derivatives possessing strong watery, floral, watermelon odor characteristics with a sparkling clean connotation. The disclosure furthermore refers to methods of their production, and to fragrance compositions containing these.

In the fragrance and flavor industry, perfumers and flavorists are continually looking for new compounds of high impact odors, or imparting new odor notes. A lot of fragrance ingredients providing floral-type odor characteristics and ingredients possessing watery, marine odor characteristics are well known. However ingredients providing a well-balanced odor profile of floral and watery are highly thought of.

2,3-Dihydro-1H-indene-2-carbaldehyde derivatives are known from the literature. For example, 2-methylindane-2-carboxaldehyde (A) is described in US patent 5 552 379 having a "metallic, aldehydic, green character, also watery and vaguely phenolic" odor.

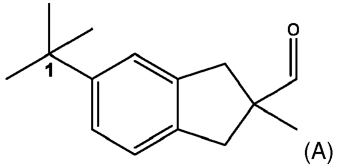

Surprisingly it was found that derivatives of (A) wherein the lipophilic part, namely the substituent tert- butyl, is replaced by - (CH₂)ₙ-CH(CH₃)₂ wherein n = 1 or 2, results in very strong watery, floral, watermelon like odorants. In particular it was found that compounds of formula (l) as defined herein below possess a remarkable low odor threshold value compared to compound (A).It was found that 2,3-dihydro-1H-indene-2-carbaldehyde derivatives becoming more powerful when the alkyl substituent of the aromatic ring is either branched at C-2 or C-3 which is really surprising.

WO 2014/180952 A1 discloses 5-isobutyl-indane-2-carbaldehyde as an odorant.

As used herein, "odor threshold value" means the lowest concentration of a vapour in the air which can be detected by smell. Generally speaking, it can be said that a compound with a low odor threshold value is more powerful than a compound with a high odor threshold value and thus allows the use of very low concentration in a fragrance composition to achieve an olfactory effect.

There is provided in a first aspect a compound of formula (I) 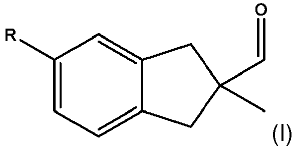 wherein R is selected from the group consisting of isopentyl (-(CH₂)₂-CH(CH₃)₂)and isobutyl (-CH₂-CH(CH₃)₂).

The compounds of formula (l) comprise one chiral center and as such may exist as a mixture of stereoisomers, or they may be resolved as isomerically pure forms. Resolving stereoisomers adds to the complexity of manufacture and purification of these compounds and so it is preferred to use the compounds as mixtures of their stereoisomers simply for economic reasons. However, if it is desired to prepare individual stereoisomers, this may be achieved according to methods known in the art, e.g. preparative HPLC and GC, crystallization or stereoselective synthesis.

As a specific example of compounds of formula (I), one may cite 5-isopentyl-2-methyl-2,3-dihydro-1H-indene-2-carbaldehyde, which possesses a very intense, floral, green, fatty, watery, aldehydic, watermelon rind odor profile. 5-Isopentyl-2-methyl-2,3-dihydro-1H-indene-2-carbaldehyde provides a very clear signal on dry fabrics even at very low dosage, such as 0.1 weight % or less based on a fragrance composition, with good substantivity. With regard to the odor profile, 5-isopentyl-2-methyl-2,3-dihydro-1H-indene-2-carbaldehyde is described by the perfumers as an improvement of Azurone^{®} (7-(3-Methylbutyl)-2H-1,5-benzodioxepin-3(4H)-one) bringing a sparkling clean, aldehydic aspect, beside the green, watery facet known from Azurone^{®}.

As a further example one my cite 5-isobutyl-2-methyl-2,3-dihydro-1H-indene-2-carbaldehyde, possessing a floral, watery, aldehydic, white floral muguet, watermelon odor profile which brings a very fresh, clean sparkling effect to a fragrance composition, and thus may be preferred over 5-isopentyl-2-methyl-2,3-dihydro-1H-indene-2-carbaldehyde, even though it is less intense compared to 5-isopentyl-2-methyl-2,3-dihydro-1H-indene-2-carbaldehyde.

The compound of formula (I) may be used alone, as stereoisomeric mixture, or in combination with a base material. As used herein, the 'base material' includes all known odorant molecules selected from the extensive range of natural products, and synthetic molecules currently available, such as essential oils, alcohols, aldehydes and ketones, ethers and acetals, esters and lactones, macrocycles and heterocycles, and/or in admixture with one or more ingredients or excipients conventionally used in conjunction with odorants in fragrance compositions, for example, carrier materials, and other auxiliary agents commonly used in the art.

The term "auxiliary agent" refers to ingredients that might be employed in a fragrance composition for reasons not specifically related to the olfactive performance of said composition. For example, an auxiliary agent may be an ingredient that acts as an aid to processing a fragrance ingredient or ingredients, or a composition containing said ingredient(s), or it may improve handling or storage of a fragrance ingredient or composition containing same. It might also be an ingredient that provides additional benefits such as imparting color or texture. It might also be an ingredient that imparts light resistance or chemical stability to one or more ingredients contained in a fragrance composition. A detailed description of the nature and type of auxiliary agents commonly used in fragrance compositions containing same cannot be exhaustive, but it has to be mentioned that said ingredients are well known to a person skilled in the art.

As used herein, 'fragrance composition' means any composition comprising the compound of formula (I) and a base material, e.g. a diluent conventionally used in conjunction with odorants, such as diethyl phthalate (DEP), dipropylene glycol (DPG), isopropyl myristate (IPM), triethyl citrate (TEC) and alcohol (e.g. ethanol). Optionally, the composition may comprise an anti-oxidant. Said anti-oxidant may be selected from Tinogard^{®} TT (BASF), Tinogard^{®} Q (BASF), Tocopherol (including its isomers, CAS 59-02-9; 364-49-8; 18920-62-2; 121854-78-2), 2,6-bis(1,1-dimethylethyl)-4-methylphenol (BHT, CAS 128-37-0) and related phenols, hydroquinones (CAS 121-31-9).

The following list comprises examples of known odorant molecules, which may be combined with the compound of the present invention:
- essential oils and extracts, e.g. castoreum, costus root oil, oak moss absolute, geranium oil, tree moss absolute, basil oil, fruit oils, such as bergamot oil and mandarine oil, myrtle oil, palmarose oil, patchouli oil, petitgrain oil, jasmine oil, rose oil, sandalwood oil, wormwood oil, lavender oil and/ or ylang-ylang oil;
- alcohols, e.g. cinnamic alcohol ((E)-3-phenylprop-2-en-1-ol); cis-3-hexenol ((Z)-hex-3-en-1-ol); citronellol (3,7-dimethyloct-6-en-1-ol); dihydro myrcenol (2,6-dimethyloct-7-en-2-ol); Ebanol^{™} ((E)-3-methyl-5-(2,2,3-trimethylcyclopent-3-en-1-yl)pent-4-en-2-ol); eugenol (4-allyl-2-methoxyphenol); ethyl linalool ((E)-3,7-dimethylnona-1,6-dien-3-ol); farnesol ((2E,6Z)-3,7,11-trimethyldodeca-2,6,10-trien-1-ol); geraniol ((E)-3,7-dimethylocta-2,6-dien-1-ol); Super Muguet^{™} ((E)-6-ethyl-3-methyloct-6-en-1-ol); linalool (3,7-dimethylocta-1,6-dien-3-ol); menthol (2-isopropyl-5-methylcyclohexanol); Nerol (3,7-dimethyl-2,6-octadien-1-ol); phenyl ethyl alcohol (2-phenylethanol); RhodinolTM (3,7-dimethyloct-6-en-1-ol); Sandalore^{™} (3-methyl-5-(2,2,3-trimethylcyclopent-3-en-1-yl)pentan-2-ol); terpineol (2-(4-methylcyclohex-3-en-1-yl)propan-2-ol); or Timberol^{™} (1-(2,2,6-trimethylcyclohexyl)hexan-3-ol); 2,4,7-trimethylocta-2,6-dien-1-ol, and/or [1-methyl-2(5-methylhex-4-en-2-yl)cyclopropyl]-methanol;
- aldehydes and ketones, e.g. anisaldehyde (4-methoxybenzaldehyde); alpha amyl cinnamic aldehyde (2-benzylideneheptanal); Georgywood^{™} (1-(1,2,8,8-tetramethyl-1,2,3,4,5,6, 7,8-octahydronaphthalen-2-yl)ethanone); Hydroxycitronellal (7-hydroxy-3,7-dimethyloctanal); Iso E Super^{®} (1-(2,3,8,8-tetramethyl-1,2,3,4,5,6,7,8-octahydronaphthalen-2-yl)ethanone); Isoraldeine^{®} ((E)-3-methyl-4-(2,6,6-trimethylcyclohex-2-en-1-yl)but-3-en-2-one); Hedione^{®} (methyl 3-oxo-2-pentylcyclopentaneacetate); 3-(4-isobutyl-2-methylphenyl)propanal; maltol; methyl cedryl ketone; methylionone; verbenone; 2,6,10-trimethylundeca-5,9-dienal; 3-(4-ethylphenyl)-2,2-dimethylpropanal ; 1-methyl-4-(4-methylpent-3-en-1-yl)cyclohex-3-ene-1-carbaldehyde; and/or vanillin;
- ether and acetals, e.g. Ambrox^{®} (3a,6,6,9a-tetramethyl-2,4,5,5a,7,8,9,9b-octahydro-1H-benzo[e][1]benzofuran); geranyl methyl ether ((2E)-1-methoxy-3,7-dimethylocta-2,6-diene); rose oxide (4-methyl-2-(2-methylprop-1-en-1-yl)tetrahydro-2H-pyran); and/ or Spirambrene^{®} (2',2',3,7,7-pentamethylspiro[bicyclo[4.1.0]heptane-2,5'-[1,3]dioxane]) ;
- esters and lactones, e.g. benzyl acetate; cedryl acetate ((1S,6R,8aR)-1,4,4,6-tetramethyloctahydro-1H-5,8a-methanoazulen-6-yl acetate); y-decalactone (6-pentyltetrahydro-2H-pyran-2-one); Helvetolide^{®} (2-(1-(3,3-dimethylcyclohexyl)ethoxy)-2-methylpropyl propionate); γ-undecalactone (5-heptyloxolan-2-one); and / or vetiveryl acetate ((4,8-dimethyl-2-propan-2-ylidene-3,3a,4,5,6,8a-hexahydro-1H-azulen-6-yl) acetate);
- macrocycles, e.g. Ambrettolide ((Z)-oxacycloheptadec-10-en-2-one); ethylene brassylate (1,4-dioxacycloheptadecane-5,17-dione); and / or Exaltolide^{®} (16-oxacyclohexadecan-1-one); and
- heterocycles, e.g. isobutylquinoline (2-isobutylquinoline).

The compound according to formula (l) may be used in a broad range of fragranced articles, e.g. in any field of fine and functional perfumery, such as perfumes, air care products, household products, laundry products, body care products and cosmetics. The compound can be employed in widely varying amounts, depending upon the specific article and on the nature and quantity of other odorant ingredients. The proportion is typically from 0.0001 to 30 weight per cent of the article. In one embodiment, the compound of the present invention may be employed in a fabric softener in an amount from 0.001 to 0.3 weight per cent. In another embodiment, the compound of the present invention may be used in fine perfumery in amounts from 0.01 to 30 weight per cent (e.g. up to about 10 or up to 20 weight per cent), more preferably between 0.01 and 5 weight per cent. However, these values are given only by way of example, since the experienced perfumer may also achieve effects or may create novel accords with lower or higher concentrations.

The compound as described hereinabove may be employed in a consumer product base simply by directly mixing the compound of formula (I), or a fragrance composition with the consumer product base, or it may, in an earlier step, be entrapped with an entrapment material, for example, polymers, capsules, microcapsules and nanocapsules, liposomes, film formers, absorbents such as carbon or zeolites, cyclic oligosaccharides and mixtures thereof, or it may be chemically bonded to substrates, which are adapted to release the fragrance molecule upon application of an external stimulus such as light, enzyme, oxygen, or the like, and then mixed with the consumer product base.

Thus, the present disclosure additionally provides a method of manufacturing a fragranced article, comprising the incorporation of a compound of formula (I), as a fragrance ingredient, either by directly admixing the compound to the consumer product base or by admixing a fragrance composition comprising the compound of formula (I), which may then be mixed with a consumer product base, using conventional techniques and methods. Through the addition of an olfactory acceptable amount of the compound of the present invention as hereinabove described the odor notes of a consumer product base will be improved, enhanced, or modified.

Thus, the invention furthermore provides a method for improving, enhancing or modifying a consumer product base by means of the addition thereto of an olfactorily acceptable amount of the compound of formula (I).

The invention also provides a fragranced article comprising:
a) as odorant the compound of formula (I), or a mixture thereof; and
b) a consumer product base.

As used herein, 'consumer product base' means a composition for use as a consumer product to fulfil specific actions, such as cleaning, softening, and caring or the like. Examples of such products include fine perfumery, e.g. perfume and eau de toilette; fabric care, household products and personal care products such as cosmetics, laundry care detergents, rinse conditioner, personal cleansing composition, detergent for dishwasher, surface cleaner; laundry products, e.g. softener, bleach, detergent; body-care products, e.g. shampoo, shower gel; air care products (includes products that contain preferably volatile and usually pleasant-smelling compounds which advantageously can even in very small amounts mask unpleasant odors). Air fresheners for living areas contain, in particular, natural and synthetic essential oils such as pine needle oils, citrus oil, eucalyptus oil, lavender oil, and the like, in amounts for example of up to 50% by weight. As aerosols they tend to contain smaller amounts of such essential oils, by way of example less than 5% or less than 2% by weight, but additionally include compounds such as acetaldehyde (in particular, <0.5% by weight), isopropyl alcohol (in particular, <5% by weight), mineral oil (in particular, <5% by weight), and propellants.

Cosmetic products include:
(a) cosmetic skincare products, especially bath products, skin washing and cleansing products, skincare products, eye makeup, lip care products, nail care products, intimate care products, foot care products;
(b) cosmetic products with specific effects, especially sunscreens, tanning products, depigmenting products, deodorants, antiperspirants, hair removers, and shaving products;
(c) cosmetic dental-care products, especially dental and oral care products, tooth care products, cleaners for dental prostheses, adhesives for dental prostheses; and
(d) cosmetic hair care products, especially hair shampoos, hair care products, hair setting products, hair-shaping products, and hair coloring products.

This list of products is given by way of illustration, and is not to be regarded as being in any way limiting.

The invention is now further described with reference to the following non-limiting examples. These examples are for the purpose of illustration only and it is understood that variations and modifications can be made by one skilled in the art.
All products were purified after work-up by either flash chromatography (FC) using *Merck* silica gel 60 (particle size 0.040-0.063 mm) or distillation. NMR spectra were measured in CDCl₃ or C₆D₆ and are reported relative to TMS (¹H NMR spectrum) or relative to CDCl₃ (¹³C NMR spectrum) as follows: chemical shifts (δ ppm), coupling constants *J* in Hz. GC-MS analyses were run on a MSD5975C mass spectrometer and are reported as m/z list (relative intensity). Odor description refers to the odor of the isomeric mixture of the compounds unless otherwise indicated.

### Example 1: 5-Isobutyl-2-methyl-2,3-dihydro-1H-indene-2-carbaldehvde

a) 6-Isobutyl-2-methyl-2,3-dihydro-1*H*-inden-1-one: To a 100 mL three-necked flask with a magnetic stirrer under argon atmosphere was added phosphoric acid (85% aqueous solution, 31.6 g, 0.274 mol). Phosphorus pentoxide (20.0 g, 0.141 mol) was added in small portions over 30 min. The mixture was heated to 180 °C and stirred for 30 min before cooled to 80 °C. Then 3-(4-isobutylphenyl)-2-methylpropanoic acid (8.00 g, 0.0363 mol) was added and then stirred for 30 min at 80 °C. The reaction mixture was cooled to room temperature and poured into 500 mL ice-water with vigorous stirring. The reaction was extracted MTBE (100 mLx2). The combined organic layer was washed successively with water (100 mLx5), saturated aqueous solution of sodium bicarbonate (30.0 mL) and brine (30.0 mL). The organic layer was dried over anhydrous magnesium sulfate. The solvent was removed by rotary evaporation and the crude product was purified by flash chromatography (iso-Hexane/MTBE = 20/1) followed by distillation through Kugelrohr (140 °C/0.1 mbar) to give 6-isobutyl-2-methyl-2,3-dihydro-1*H*-inden-1-one (3.93 g, 19.4 mmol, 49% yield) as a colorless liquid.
b) Methyl 6-isobutyl-2-methyl-1-oxo-2,3-dihydro-1H-indene-2-carboxylate: To a solution of diisopropylamine (20.9 mL, 148 mmol, 1.5 equiv) in THF (100 mL) was added dropwise at -70 °C *n*-BuLi (80 mL, 129 mmol, 1.3 equiv, 1.6 M in hexanes) over 30 min. After stirring the LDA solution for 30 min at -70°C, a solution of 6-isobutyl-2-methyl-2,3-dihydro-1H-inden-1-one (20.0 g, 98.9 mmol, 1.0 equiv,) in THF (70 mL) was added dropwise at -70 °C over 45 min. The color of the reaction mixture changed from yellow to brown. After stirring for 15 min at -70 °C, a solution of methyl carbonocyanidate (12.7 g, 148 mmol, 1.5 equiv) in THF (70 mL) was added dropwise over 15 min. During the addition, the color of the reaction mixture changed to purple. The reaction mixture was allowed to warm to room temperature over 2 h. Since the starting material was fully consumed, the reaction mixture was quenched at 0 °C with sat. NH₄Cl solution and diluted with MTBE and water. The organic layer was separated and washed with sat. NH₄Cl-solution (1 x), water (1 x) and brine (2 x). All aqueous layers were back extracted once with MTBE. The combined organic extracts were dried over MgSO₄, filtered and concentrated under reduced pressure to give a brown liquid (28.7 g). The crude product was distilled over a 3 cm Vigreux column (bp 140-142 °C at 0.05 mbar) to give methyl 6-isobutyl-2-methyl-1-oxo-2,3-dihydro-1H-indene-2-carboxylate (22.1 g, 86% yield) as an orange liquid. Odor description: odorless.
   ¹H NMR (400 MHz, CDCl₃): δ 7.56 (s, 1H), 7.43 (dd, J=7.8, 1.7 Hz, 1H), 7.31 (dd, J=7.8, 0.7 Hz, 1H), 3.68 (s, 3H), 3.67 (d, J=16.6 Hz, 1H), 2.95 (d, J=17.1 Hz, 1H), 2.53 (d, J=7.3 Hz, 2H), 1.88 (m_{c}, 1H), 1.52 (s, 3H), 0.91 (d, J=6.6 Hz, 6H) ppm. ¹³C NMR (101 MHz, CDCl₃): δ 203.6, 172.6, 150.2, 141.7, 136.7, 134.7, 126.0, 124.9, 56.3, 52.6, 44.9, 39.7, 30.2, 22.2, 21.2 ppm. GC-MS (EI) m/z (%): 260 (37) [M⁺], 201 (52), 200 (72), 159 (12), 158 (50), 157 (100), 129 (29), 128 (36), 127 (14), 115 (37). bp 140-142 °C (0.05 mbar).
c) Methyl 5-isobutyl-2-methyl-2,3-dihydro-1H-indene-2-carboxylate: 15 pressure vials were charged with 140-150 mg of 5% Pd/C each (in total 2.2 g, 10% wt/wt 5% Pd/C relative to the substrate). Then, methyl 6-isobutyl-2-methyl-1-oxo-2,3-dihydro-1H-indene-2-carboxylate (22.0 g, 84.5 mmol, 1.0 equiv) was dissolved in acetic acid (220 mL) and concentrated sulfuric acid (22 mL). This stock solution was split up in 15 portions and was filled into the prepared vials. Each vial was equipped with a magnetic stirring bar, closed and pressurized with H₂ (50 bar) at room temperature for 3 h. All reaction mixtures were combined and filtered through a plug of Celite. The filter cake was washed with MTBE and the filtrate was washed with water (3 x), sat. NaHCO₃ (1 x) and brine (1 x). All aqueous layers were back extracted once with MTBE. The combined organic extracts were dried over MgSO₄, filtered and concentrated under reduced pressure to give a yellow liquid (20.1 g). The crude was distilled over a 10 cm Vigreux column (bp 98-99 °C at 0.05 mbar) to afford methyl 5-isobutyl-2-methyl-2,3-dihydro-1H-indene-2-carboxylate (14.3 g, 69% yield) as a colorless liquid.
   ¹H NMR (400 MHz, CDCl₃): δ 7.08 (d, J=7.8 Hz, 1H), 6.97 (s, 1H), 6.93 (d, J=7.8 Hz, 1H), 3.71 (s, 3H), 3.45 (dd, J=16.0, 6.5 Hz, 2H), 2.78 (d, J=15.7 Hz, 2H), 2.43 (d, J=7.3 Hz, 2H), 1.83 (m_{c}, 1H), 1.36 (s, 3H), 0.90 (d, J=6.6 Hz, 6H) ppm. ¹³C NMR (101 MHz, CDCl₃): δ 178.2, 141.1, 140.1, 138.4, 127.4, 125.3, 124.1, 52.0, 49.6, 45.3, 43.9, 43.7, 30.3, 25.1, 22.4 ppm. IR (neat): 2952, 2928, 2868, 1732, 1493, 1462, 1435, 1305, 1261, 1230, 1201, 1168, 1112, 1084, 994, 892, 833, 805, 791, 771 cm⁻¹. HRMS (ESI+) m/z calc'd for C₁₆H₂₂O₂ [M+H]⁺: 247.1693, found 247.1690. GC-MS (EI) m/z (%): 246 (12) [M⁺], 203 (11), 187 (16), 186 (43), 144 (19), 143 (100), 129 (21), 128 (32), 127 (74), 115 (75). bp 98-99 °C (0.05 mbar).
   Odor description: cooked vegetables, then very weak, green.
d) (5-lsobutyl-2-methyl-2,3-dihydro-1H-inden-2-yl)methanol: To a suspension of LiAlH₄ (1.43 g, 37.7 mmol, 0.8 equiv) in Et₂O (150 mL) was added dropwise at -5 °C a solution of methyl 5-isobutyl-2-methyl-2,3-dihydro-1H-indene-2-carboxylate (11.6 g, 47.1 mmol, 1.0 equiv) in Et₂O (25 mL) over 25 min. After stirring for 10 min at -5 °C, the reaction mixture was quenched with water (1.5 mL), 15% NaOH (1.5 mL) and water (3 mL). After stirring for 1.5 h at room temperature, the white suspension was filtered and the filter cake was rinsed thoroughly with MTBE. The filtrate was concentrated under reduced pressure to give (5-isobutyl-2-methyl-2,3-dihydro-1H-inden-2-yl)methanol (9.75 g, 95%) as a colorless liquid, which was used in the next step without further purification. An analytical sample was obtained by Kugelrohr distillation (165 °C, 0.05 mbar). Odor description: odorless.
   ¹H NMR (400 MHz, CDCl₃): δ 7.07 (d, J=7.8 Hz, 1H), 6.96 (s, 1H), 6.91 (d, J=7.4 Hz, 1H), 3.53 (s, 2H), 2.88 (dd, J=15.9, 5.9 Hz, 2H), 2.64 (dd, J=16.6, 1.2 Hz, 2H), 2.43 (d, J=7.1 Hz, 2H), 1.83 (m_{c}, 1H), 1.50 (br s, 1H), 1.18 (s, 3H), 0.90 (d, J=6.6 Hz, 6H) ppm. ¹³C NMR (101 MHz, CDCl₃): δ 142.4, 139.8, 139.6, 127.1, 125.5, 124.3, 70.7, 45.3, 45.1, 42.7, 42.4, 30.4, 24.1, 22.4 ppm. GC-MS (EI) m/z (%): 218 (21) [M⁺], 175 (41), 158 (14), 157 (100), 143 (27), 142 (26), 141 (19), 129 (46), 128 (49), 115 (24).
e) 5-Isobutyl-2-methyl-2,3-dihydro-1H-indene-2-carbaldehyde: A round-bottomed flask was charged with (5-isobutyl-2-methyl-2,3-dihydro-1H-inden-2-yl)methanol (9.75 g, 44.7 mmol, 1.0 equiv), pyridinium chlorochromate (10.1 g, 46.9 mmol, 1.05 equiv), Celite (10.2 g) and CH₂Cl₂ (200 mL). After stirring the brown suspension at room temperature overnight (16 h), the reaction mixture was filtered over a plug of Celite and the filter cake was washed thoroughly with CH₂Cl₂. The filtrate was concentrated under reduced pressure to give a black suspension (11.7 g). The crude was purified by flash chromatography (hexane/MTBE, 9:1) and Kugelrohr distillation (160 °C at 0.05mbar) to afford 5-isobutyl-2-methyl-2,3-dihydro-1H-indene-2-carbaldehyde (7.6 g, 79% yield over 2 steps) as a colorless liquid.
   ¹H NMR (400 MHz, CDCl₃): δ 9.66 (s, 1H), 7.10 (d, J=7.8 Hz, 1H), 6.99 (s, 1H), 6.95 (d, J=7.5 Hz, 1H), 3.34 (dd, J=16.0, 5.0 Hz, 2H), 2.74 (d, J=15.7 Hz, 2H), 2.44 (d, J=7.3 Hz, 2H), 1.84 (m_{c}, 1H), 1.30 (s, 3H), 0.91 (d, J=6.6 Hz, 6H) ppm. ¹³C NMR (101 MHz, CDCl₃): δ 204.1, 140.8, 140.4, 138.1, 127.7, 125.4, 124.2, 54.4, 45.3, 40.8, 40.6, 30.3, 22.4, 21.0 ppm. IR (neat): 2953, 2926, 2868, 2845, 1726, 1702, 1493, 1463, 1436, 1383, 1366, 1230, 1184, 1168, 939, 918, 885, 833, 805, 758 cm⁻¹. HRMS (ESI+) m/z calc'd for C₁₅H₂₀O [M+Na]⁺: 239.1406, found 239.1403. GC-MS (EI) m/z (%): 216 (26) [M⁺], 201 (26), 173 (56), 159 (27), 145 (25), 131 (19), 129 (100), 128 (67), 127 (20), 115 (28).
   Odor description: floral, watery, aldehydic, white floral muguet, watermelon, intense.

### Example 2: 5-Isopentyl-2-methyl-2,3-dihydro-1H-indene-2-carbaldehyde

a) Methyl 2-methyl-5-(3-methylbutanoyl)-2,3-dihydro-1H-indene-2-carboxylate: To a solution of methyl 2-methyl-2,3-dihydro-1H-indene-2-carboxylate (20.0 g, 105 mmol, 1.0 equiv, prepared according to WO2008021849) in CH₂Cl₂ (350 ml) was added at 0 °C AlCl₃ (28.0 g, 210 mmol, 2.0 equiv) in small portions. After stirring the orange reaction mixture for 1 h at 0°C, methylbutanoyl chloride (20.0 mL, 164 mmol, 1.55 equiv) was added dropwise at that temperature over 10 min. The brown reaction mixture was allowed to warm to room temperature in the ice bath overnight (16 h). After cooling to 0 °C, the reaction mixture was quenched slowly with 2N HCI, while keeping the internal temperature below 15 °C. The organic layer was separated and washed with water (2 x), sat. NaHCO₃ (1 x) and brine (1 x). All aqueous layers were back extracted once with CH₂Cl₂. The combined organic extracts were dried over MgSO₄, filtered and concentrated under reduced pressure to give a brown liquid (28.1 g). The crude was purified twice by flash chromatography (hexane/MTBE, 4:1) to give methyl 2-methyl-5-(3-methylbutanoyl)-2,3-dihydro-1H-indene-2-carboxylate as an orange liquid (20.3 g, 70%). Odor description: odorless.
   ¹H NMR (400 MHz, CDCl₃): δ 7.78 (s, 1H), 7.77 (d, J=8.0 Hz, 1H), 7.26 (d, J=8.2 Hz, 1H), 3.72 (s, 3H), 3.51 (dd, J=16.5, 4.3 Hz, 2H), 2.87 (dd, J=16.3, 5.7 Hz, 2H), 2.81 (d, J=6.8 Hz, 2H), 2.29 (m_{c}, 1H), 1.37 (s, 3H), 0.99 (d, J=6.6 Hz, 6H). ¹³C NMR (101 MHz, CDCl₃): δ 200.0, 177.5, 146.9, 141.9, 136.4, 127.1, 124.6, 124.3, 52.2, 49.8, 47.5, 43.9, 43.7, 25.2, 24.9, 22.7 ppm. GC-MS (EI) m/z (%): 274 (12) [M⁺], 232 (14), 218 (14), 217(100), 215 (12), 214 (14), 199 (61), 172 (23), 157 (57), 143 (15), 130 (10), 129 (43), 128 (49), 127 (17), 115 (25).
b) Methyl 5-isopentyl-2-methyl-2,3-dihydro-1H-indene-2-carboxylate: 4 pressure vials were charged with 175 mg of 5% Pd/C each (in total 700 mg, 10% wt/wt 5% Pd/C relative to the substrate). Then, a stock solution of methyl methyl 2-methyl-5-(3-methylbutanoyl)-2,3-dihydro-1H-indene-2-carboxylate (7.15 g, 26.1 mmol, 1.0 equiv) in acetic acid (70 mL) and concentrated sulfuric acid (7 mL) was prepared, split up in 4 portions and was charged into the previously prepared vials. Each vial was equipped with a magnetic stirring bar, closed and pressurized with H₂ (50 bar) at room temperature for 3 h. All reaction mixtures were combined and filtered through a plug of Celite. The filter cake was washed with MTBE and the filtrate was washed water (3 x), sat. NaHCO₃ (1 x) and brine (1 x). All aqueous layers were back extracted once with MTBE. The combined organic extracts were dried over MgSO₄, filtered and concentrated under reduced pressure to give a yellow liquid (6.74 g). The crude was purified by flash chromatography (hexane/MTBE, 4:1) to give methyl 5-isopentyl-2-methyl-2,3-dihydro-1H-indene-2-carboxylate (3.5 g, 52% yield) as a colorless liquid. Odor description: odorless.
   ¹H NMR (400 MHz, CDCl₃): δ 7.09 (d, J=7.6 Hz, 1H), 7.02 (s, 1H), 6.98 (d, J=7.5 Hz, 1H), 3.72 (s, 3H), 3.45 (dd, J=15.8, 7.5 Hz, 2H), 2.78 (d, J=15.7 Hz, 2H), 2.52-2.61 (m, 2H), 1.65-1.53 (m, 1H), 1.44-1.53 (m, 2H), 1.36 (s, 3H), 0.94 (d, J=6.6 Hz, 6H) ppm. ¹³C NMR (101 MHz, CDCl₃): δ 178.2, 141.5, 141.3, 138.3, 126.7, 124.6, 124.3, 52.0, 49.6, 43.9, 43.7, 41.2, 33.6, 27.7, 25.1, 22.5 ppm. IR (neat): 2952, 2932, 2869, 1732, 1493, 1461, 1434, 1367, 1303, 1258, 1229, 1200, 1268, 1112, 1085, 994, 890, 815, 791, 770 cm⁻¹. HRMS (ESI+) m/z calc'd for C₁₇H₂₄O₂ [M+H]⁺: 261.1849, found 261.1846. GC-MS (EI) m/z (%): 260 (18), 201 (18), 200 (44), 145 (18), 144 (100), 143 (92), 141 (14), 129 (44), 128 (53), 115 (15).
c) (5-Isopentyl-2-methyl-2,3-dihydro-1H-inden-2-yl)methanol: To a suspension of LiAlH₄ (0.38 g, 9.68 mmol, 0.8 equiv) in Et₂O (50 mL) was added dropwise at -5 °C a solution of methyl 5-isopentyl-2-methyl-2,3-dihydro-1H-indene-2-carboxylate (3.15 g, 12.1 mmol, 1.0 equiv) in Et₂O (10 mL) over 10 min. After stirring for 15 min at -5 °C, the reaction mixture was quenched with water (0.38 mL), 15% NaOH (0.76 mL) and water (0.38 mL). After stirring for 4 h at room temperature, the white suspension was filtered and the filter cake was rinsed thoroughly with MTBE. The filtrate was concentrated under reduced pressure. The crude (2.82 g) was purified by flash chromatography (hexane/MTBE, 1:1) to give (5-isopentyl-2-methyl-2,3-dihydro-1H-inden-2-yl)methanol (2.62 g, 93% yield) as a colorless liquid. Odor description: odorless.
   ¹H NMR (400 MHz, CDCl₃): δ 7.07 (d, J=7.6 Hz, 1H), 7.00 (s, 1H), 6.95 (d, J=7.9 Hz, 1H), 3.53 (d, J=5.6 Hz, 2H), 2.88 (dd, J=15.8, 6.7 Hz, 2H), 2.63 (dd, J=15.9, 3.4 Hz, 2H), 2.51-2.60 (m, 2H), 1.53-1.63 (m, 1H), 1.42-1.53 (m, 3H), 1.18 (s, 3H), 0.93 (d, J=6.6 Hz, 6H) ppm. ¹³C NMR (101 MHz, CDCl₃): δ 142.6, 141.2, 139.6, 126.4, 124.8, 124.5, 70.8, 45.0, 42.7, 42.4, 41.2, 33.7, 27.7, 24.1, 22.6 ppm. GC-MS (EI) m/z (%): 232 (32) [M⁺], 158 (96), 157 (89), 143 (84), 142 (33), 141 (32), 129 (100), 128 (75), 115 (35), 43 (24).
d) 5-Isopentyl-2-methyl-2,3-dihydro-1H-indene-2-carbaldehyde: A round-bottomed flask was charged (5-isopentyl-2-methyl-2,3-dihydro-1H-inden-2-yl)methanol (2.35 g, 10.1 mmol, 1.0 equiv), pyridinium chlorochromate (2.34 g, 10.6 mmol, 1.05 equiv), Celite (2.34 g) and CH₂Cl₂ (50 mL). After stirring the brown suspension at room temperature overnight (18 h), the reaction mixture was filtered over a plug of Celite and the filter cake was washed thoroughly with CH₂Cl₂ (3 x 20 mL). The filtrate was concentrated under reduced pressure to give a brown liquid (3.56 g). The crude was purified by flash chromatography (hexane/MTBE, 9:1) and Kugelrohr distillation (155 °C at 0.06 mbar) to afford 5-isopentyl-2-methyl-2,3-dihydro-1H-indene-2-carbaldehyde (0.96 g, 41% yield) as a colorless oil.
   ¹H NMR (400 MHz, CDCl₃): δ 9.65 (s, 1H), 7.11 (d, J=7.8 Hz, 1H), 7.04 (s, 1H), 6.99 (d, J=7.8 Hz, 1H), 3.33 (dd, J=16.1, 6.1 Hz, 2H), 2.74 (d, J=15.7 Hz, 2H), 2.54-2.61 (m, 2H), 1.60 (m_{c}, 1H), 1.41-1.53 (m, 2H), 1.30 (s, 3H), 0.94 (d, J=6.6 Hz, 6H) ppm. ¹³C NMR (101 MHz, CDCl₃): δ 204.1, 141.9, 141.1, 138.0, 127.0, 124.6, 124.4, 54.4, 41.1, 40.8, 40.6, 33.6, 27.7, 22.5, 21.0 ppm. GC-MS (EI) m/z (%): 230 (45) [M⁺], 215 (44), 173 (49), 159 (67), 131 (33), 130 (47), 129 (100), 128 (93), 115 (41), 43 (34).
   Odor description: floral, green, fatty, watery, aldehydic, watermelon rind, very intense.

### Comparison Example 3: 2-Methyl-5-(4-methylpentyl)-2,3-dihydro-1H-indene-2-carbaldehyde

The title compound was prepared, following the general procedure as described in Example 2, from methyl 2-methyl-2,3-dihydro-1H-indene-2-carboxylate and 4-methylpentanoyl chloride in 16 % yield over 4 steps affording a colorless oil.
¹H NMR (400 MHz, CDCl₃): δ 9.65 (s, 1H), 7.11 (d, J=8.1 Hz, 1H), 7.03 (s, 1H), 6.99 (d, J=7.6 Hz, 1H), 3.33 (dd, J=16.1, 6.1 Hz, 2H), 2.74 (dd, J=16.8, 1.3 Hz, 2H), 2.55 (m_{c}, 2H), 1.52-1.64 (m, 3H), 1.30 (s, 3H), 1.20-1.28 (m, 2H), 0.88 ppm (d, J=6.6 Hz, 6H). ¹³C NMR (101 MHz, CDCl₃): δ 204.1, 141.8, 141.0, 138.1, 127.0, 124.7, 124.4, 54.4, 40.8, 40.6, 38.7, 36.1, 29.6, 27.9, 22.6, 21.0 ppm. IR (neat): 2953, 2928, 2868, 1726, 1701, 1493, 1461, 1436, 1384, 1366, 1230, 1183, 1124, 1063, 939, 909, 882, 821, 776, 704 cm⁻¹. HRMS (ESI+) m/z calc'd for C₁₇H₂₄O [M+Na]⁺: 267.1719, found 267.1717. GC-MS (EI) m/z (%): 244 (45) [M⁺], 229 (43), 173 (54), 159 (56), 129 (100); 128 (76), 115 (31), 43 (95), 41 (40), 29 (23).
Odor description: aldehydic, green, metallic, watery, swimming pool, floralozone.

### Comparison Example 4: 5-(sec-Butyl)-2-methyl-2,3-dihydro-1H-indene-2-carbaldehyde

The title compound was prepared, following the general procedure as described in Example 1, starting from 6-(*sec*-butyl)-2-methyl-2,3-dihydro-1H-inden-1-one using a four step sequence giving a colorless liquid.
¹H NMR (400 MHz, CDCl₃): δ 9.66 (s, 1H), 7.12 (d, J=7.8 Hz, 1H), 7.03 (s, 1H), 7.00 (d, J=7.6 Hz, 1H), 3.35 (dd, J=16.0, 7.0 Hz, 2H), 2.75 (dd, J=16.0, 3.8 Hz, 2H), 2.57 (m_{c}, 1H), 1.52-1.64 (m, 2H), 1.31 (s, 3H), 1.23 (d, J=7.1 Hz, 3H), 0.84 (t, J=7.3 Hz, 3H) ppm. ¹³C NMR (101 MHz, CDCl₃, 1:1 mixture of 2 diastereoisomers): δ 204.1, 146.6, 141.0, 138.2, 125.7 (2x), 124.4, 123.3 (2x), 54.3, 41.5, 40.9, 40.6, 31.3 (2x), 22.0 (2x), 21.0, 12.3 ppm. IR (neat): 2960, 2928, 2872, 1725, 1492, 1456, 1435, 1374, 1234, 1183, 1120, 1063, 958, 939, 913, 883, 821, 776, 716, 585 cm⁻¹. GC-MS (EI) m/z (%): 216 (34) [M⁺], 187 (100), 159 (32), 145 (31), 143 (60), 141 (26), 129 (35), 128 (66), 115 (39), 29 (53).
Odor description: aldehydic, green, fatty, rubbery, bourgeonal.

### Example 5: Determination of GC-odor threshold values

According to standard procedures known to the person skilled in the art, threshold values for volatile perfumery compounds are determined on a gas chromatograph equipped with a sniff port by a panel of trained evaluators. The lowest concentration smelled by each panelist is recorded as the individual threshold value expressed in ng (absolute amount of compound delivered at the sniff port).

Under identical conditions the odor threshold value for the individual compounds was measured. The results are given below.

| Compound of formula (I) | Odor threshold concentration (nq/L) |
|---|---|
| a: R= tert. butyl (comparison; compound A) | 4.2 |
| b: R = isobutyl | 0.14 |
| c: R = 3-methyl butyl | 0.018 |
| d: R = 4-methyl pentyl (comparison) | 0.3 |
| e: R = but-2-yl (comparison) | 1.3 |

As can be seen from the results above, the odor threshold concentration of the compounds of formula (l) wherein R is isobutyl is 30 times lower compared to compound A.

| Compound of formula (lb) | Odor threshold concentration (nq/L) |
|---|---|
| R' = methyl | 0.14 |
| R' = hydrogen (5-Isobutyl-2,3-dihydro-1H-indene-2-carbaldehyde - comparison) | 1.3 |

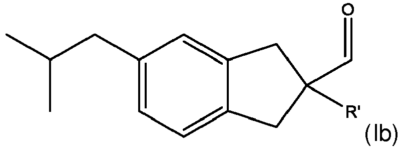

### Example 6: fragrance composition for fine perfumery

| Ingredient (parts by weight 1/1000) | composition A | composition B | composition C |
|---|---|---|---|
| Bulgarie Rose essential oil | 1.0 | 1.0 | 1.0 |
| Bergamot essential oil | 15.0 | 15.0 | 15.0 |
| Benzyl acetate | 100.0 | 100.0 | 100.0 |
| Citronellol acetate | 60.0 | 60.0 | 60.0 |
| 2-Phenylethyl acetate | 3.0 | 3.0 | 3.0 |
| 2-Phenyl-1-ethanol | 250.0 | 250.0 | 250.0 |
| 2-(Phenylmethylene)octanal | 30.0 | 30.0 | 30.0 |
| Ylang Ylang essential oil | 25.0 | 25.0 | 25.0 |
| Cyclamen aldehyde ¹⁾ | 13.0 | 13.0 | 13.0 |
| 2-phenylacetaldehyde | 0.2 | 0.2 | 0.2 |
| Citronellol | 60.0 | 60.0 | 60.0 |
| Cumarine | 2.0 | 2.0 | 2.0 |
| Cyclohexal | 100.0 | 100.0 | 100.0 |
| Ethyl linalol | 30.0 | 30.0 | 30.0 |
| Gardenol | 2.0 | 2.0 | 2.0 |
| Geranium essential oil | 2.0 | 2.0 | 2.0 |
| Hedione | 120.0 | 120.0 | 120.0 |
| Hexenol-3-cis | 7.0 | 7.0 | 7.0 |
| Rose absolute | 3.0 | 3.0 | 3.0 |
| Hydroxycitronellal | 100.0 | 100.0 | 100.0 |
| Indole | 15.0 | 15.0 | 15.0 |
| Jasmin absolute | 6.0 | 6.0 | 6.0 |
| Cis-Jasmone | 15.0 | 15.0 | 15.0 |
| Evernyl ²⁾ | 1.0 | 1.0 | 1.0 |
| Peach Pure ³⁾ | 0.3 | 0.3 | 0.3 |
| Diethyle phtalate ⁴⁾ | 1.8 | 1.8 | 1.8 |
| Virdine ((2,2-dimethoxyethyl)benzene) | 25.0 | 25.0 | 25.0 |
| Dipropylene glycol (DPG) | 12.7 | 7.7 | 2.7 |
| 5-lsobutyl-2-methyl-2,3-dihydro-1H-indene-2-carbaldehyde | - | 5 | - |
| 5-Isopentyl-2-methyl-2,3-dihydro-1H-indene-2-carbaldehyde @1% DPG | - | - | 10 |
| Total | 1000 | 1000 | 1000 |

| | | | |
|---|---|---|---|
| 1) 3-(4-isopropylphenyl)-2-methylpropanal 2) methyl 2,4-dihydroxy-3,6-dimethylbenzoate 3) 5-heptyldihydrofuran-2(3H)-one 4) diethyl 1,2-benzenedicarboxylate | | | |

All compositions are fine fragrance accords to be assessed @ 10% in alcohol 85°. The character of composition A is mainly floral, muguet, with green, rosy and jasmine undertones.
Composition B: With the addition of 0.5 weight % of 5-isobutyl-2-methyl-2,3-dihydro-1H-indene-2-carbaldehyde to Composition A, the overall character becomes more vibrant, lively, watery.
Composition C: with addition of 1.0 weight % of 5-lsopentyl-2-methyl-2,3-dihydro-1H-indene-2-carbaldehyde @1% DPG, the overall character is greener, more watery, ozonic.

## Claims

1. A compound of formula (l) in the form of any one of its stereoisomers or a mixture thereof 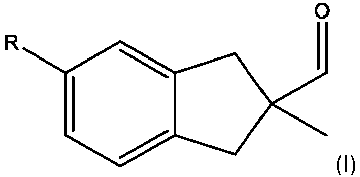 wherein R is selected from the group consisting of isopentyl and isobutyl.

2. A fragranced article comprising as odorant a compound of formula (l) as defined in claim 1, or a mixture thereof, and a consumer product base.

3. A fragranced article according to claim 2 wherein the article is a consumer product base selected from fine fragrance, household products, laundry products, body care products, cosmetic products and air care products.

4. The use as fragrance or flavour of a compound of formula (l) as defined in claim 1.

5. A method of improving, enhancing or modifying a consumer product base by means of addition thereto of an olfactory acceptable amount of a compound of formula (l) as defined in claim 1.

## Patentansprüche

1. Verbindung der Formel (I) in Form eines ihrer Stereoisomere oder eines Gemischs davon 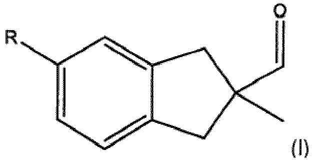 wobei R aus der Gruppe bestehend aus Isopentyl und Isobutyl ausgewählt ist.

2. Duftstoffhaltiger Artikel, umfassend eine Verbindung der Formel (I) gemäß Anspruch 1 oder ein Gemisch davon als Riechstoff und eine Konsumproduktbasis.

3. Duftstoffhaltiger Artikel nach Anspruch 2, wobei es sich bei dem Artikel um eine Konsumproduktbasis handelt, die aus Feinduftstoffen (Parfüm), Haushaltsprodukten, Waschmittelprodukten, Körperpflegeprodukten, Kosmetikprodukten und Luftbehandlungsprodukten ausgewählt ist.

4. Verwendung einer Verbindung der Formel (I) gemäß Anspruch 1 als Duftstoff oder Geschmacksstoff.

5. Verfahren zur Verbesserung, Verstärkung oder Modifizierung einer Konsumproduktbasis durch Zugabe einer olfaktorisch annehmbaren Menge einer Verbindung der Formel (I) gemäß Anspruch 1.

## Revendications

1. Composé de formule (I) sous la forme de l'un quelconque parmi ses stéréoisomères ou un mélange correspondant 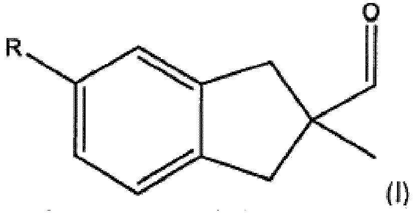 R étant choisi dans le groupe constitué par isopentyle et isobutyle.

2. Article parfumé comprenant comme substance odorante un composé de formule (I) tel que défini dans la revendication 1, ou un mélange correspondant, et une base de produit de consommation.

3. Article parfumé selon la revendication 2, l'article étant une base de produit de consommation choisie parmi un parfum fin, des produits ménagers, des produits de blanchisserie, des produits de soin personnel, des produits cosmétiques et des produits d'assainissement de l'air.

4. Utilisation en tant que parfum ou arôme d'un composé de formule (I) tel que défini dans la revendication 1.

5. Procédé d'amélioration, d'augmentation ou de modification d'une base de produit de consommation au moyen d'un ajout à celle-ci d'une quantité acceptable sur le plan olfactif d'un composé de formule (I) tel que défini dans la revendication 1.
